# EUROPEAN PATENT APPLICATION

(11) **EP 2 857 998 A1**
(43) Date of publication of application: **08.04.2015**
(21) Application number: 13187520.5
(22) Date of filing: 07.10.2013
(51) Int. Cl.: G06F 19/00

(54) **Method of formalizing temporal aspects in events of an electronic patient record**

(71) Applicant: Agfa Healthcare, 2640 Mortsel (BE)
(72) Inventor: Sun, Hong, 2640 Mortsel (BE); De Roo, Jos, 2640 Mortsel (BE); Depraetere, Kristof, 2640 Mortsel (BE); Colaert, Dirk, 2640 Mortsel (BE)
(74) Representative: Verbrugghe, Anne Marie L.

(57) **Abstract**

Data regarding at least one event are extracted from a data repository and subjected to a syntactic transformation into a first semantic representation if needed. Extracted and transformed data are converted into a representation with explicit semantics regarding temporal information. If the extracted data comprise no explicit temporal data, temporal data are deduced from other extracted data pertaining to the event and attached to the event data. The results can be applied for temporal reasoning.

## Description

### FIELD OF THE INVENTION

The present invention relates to a method of formalizing temporal aspects in events of an electronic health record (EHR).

### BACKGROUND OF THE INVENTION

Clinical decision making is a process that assists health professionals in decision making tasks such as making a diagnosis of a patient's health condition.

In a healthcare environment such a decision can be deduced from data available in a single electronic patient record of a patient. It can also be based on interpreted information originating from large sets of electronic patent records stored in different forms and formats in different data repositories.

In order to adequately serve as a basis for decisions, temporal aspects of such data from patient records is of ultimate importance. As these data are used e.g. for comparison and/or statistics and deduction of data, it is crucial to have accurate knowledge of the temporal aspects (date information) of the used data.

However, data in the electronic patient records do not always have accurate or complete temporal information.

Temporal data may be entirely missing or may be incomplete. Sometimes explicit temporal information is not given but is implicitly present and could be deduced from other data available in the electronic patient record.

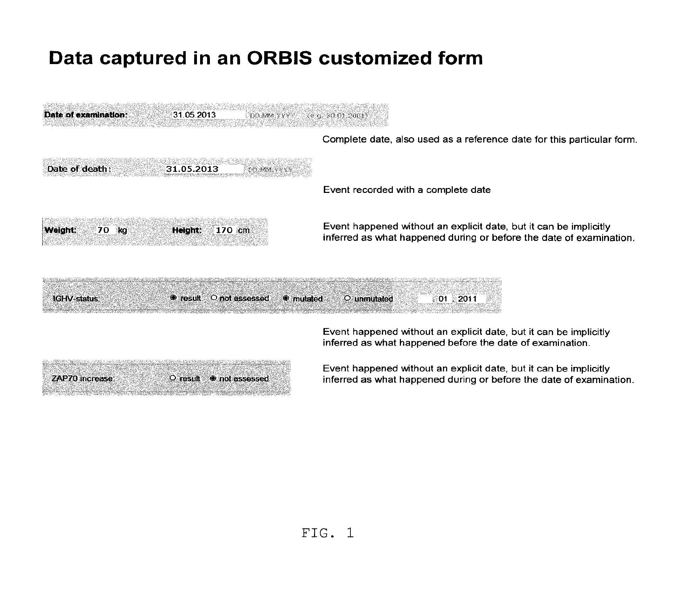

Events documented in an electronic patient record may thus have information on the date of the event in several ways. Figure 1 discloses a form in which data regarding an examination are recorded. This form is a form used in Agfa HealthCare's Clinical Information Management System, marketed under the trade name ORBIS.

Some events have a very specific date, e.g. complete data are available on the date of the examination. The examination in this example was performed on May 31, 2013. This date is used as reference for this particular form.

Furthermore, the record comprises very specific data on the date of death of the patient. The patient in this example died on May 31, 2013.

Other events may have no temporal information explicitly recorded. For example in the form shown in figure 1, the patient's weight and height are recorded. No explicit data are however available on the date on which these data were gathered. For this event without explicit date, one can infer that the weight and height are measured before or on the date of the examination.

In any case, if it is desired to be able to use the data for clinical decision making, there is a need to have this temporal information in a format that makes comparison of data from different sources and / or originally represented in different formats, possible.

### SUMMARY OF THE INVENTION

The above-mentioned advantageous aspects are realised by a method as set out in the appended independent claim.

Further embodiments of the invention are set out in the dependent claims.

The present invention relates to extraction of data from at least one event in at least one electronic patient record from at least one data repository for the purpose of further processing of these data, e.g. in a clinical decision making application or statistics.

In the context of the present invention the term 'event' refers to an item (record, and to the data relating to such an event) which has been stored in an electronic patient record. Examples of such events are: an examination, laboratory data ... or general administrative data of a patient. Other types of data may be envisaged.

The invention specifically relates to temporal aspects of these events. As has been mentioned, such temporal aspects play an important role when data comparison is concerned or when decisions are made on the basis of these data.

Temporal data (date information such as date of an examination, date of a medication etc.) may be provided explicit and complete or explicit and incomplete, or may even be absent.

The invention provides a method in which the temporal data information is formalized so that it can be used in further applications.

Further advantages and embodiments of the present invention will become apparent from the following description and drawings.

### BRIEF DESCRIPTION OF THE DRAWINGS

Figure 1 shows data regarding an event extracted from an electronic patient record,
Figure 2 schematically shows the data flow,
Figure 3 is a schematic representation of a data warehouse.

### DETAILED DESCRIPTION OF THE INVENTION

While the present invention will hereinafter be described in connection with preferred embodiments thereof, it will be understood that it is not intended to limit the invention to those embodiments.

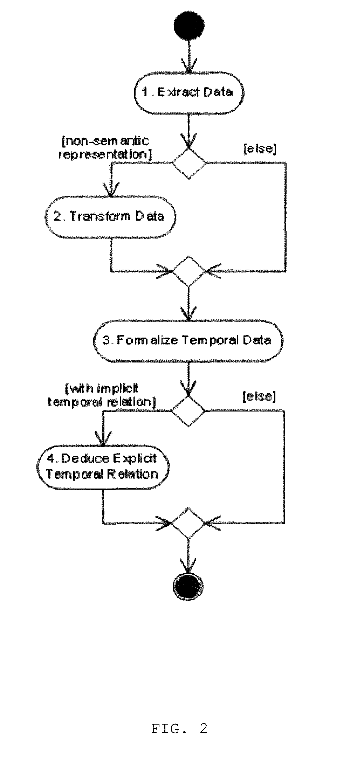

For the following explanation reference is made to figure 2 which illustrates the different steps of the present invention.

According to the present invention in a first step data concerning an event(s) is retrieved from a data repository. The retrieved data may comprise explicit temporal information (complete or partial) or no explicit temporal data of the event.

In one embodiment the data are retrieved directly from one or more database(s) such as relational data bases.

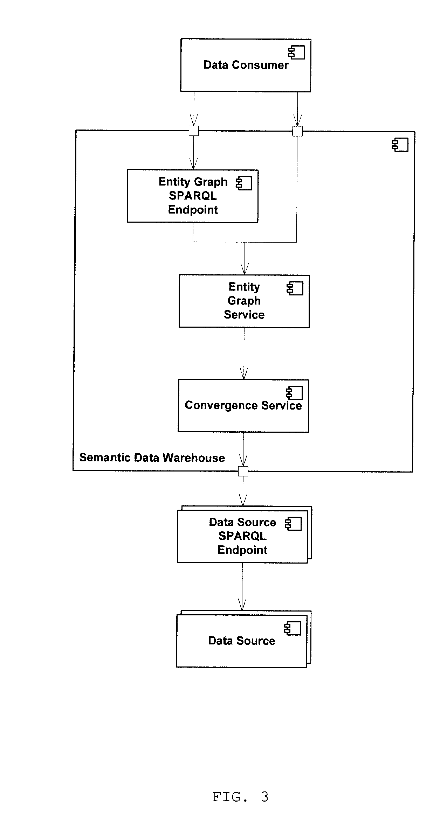

In another embodiment the data are retrieved from a data warehouse such as the data warehouse described in co-pending un-published European patent application 12182778.6 filed September 3, 2012 and entitled ' On demand semantic data warehouse'. Figure 3 is a schematic representation illustrating the composition of such a data warehouse. Data concerning stored entities become available at a SPARQL endpoint of the data warehouse.

A semantic data warehouse as described in this patent application typically comprises a convergence service for executing queries to connected data sources, converting data from source to domain semantics and aggregating converted data. The convergence service is invoked by an entity graph service. This entity graph service on demand defines a semantic entity representation, the needed queries and data sources to be queried. It projects and makes available the resulting data in said semantic entity representation.

An entity representation is stated in RDF (Resource Description Framework).
The entity representation is provided by means of a named entity graph and can be denoted by an URL.

A convergence service is a software system designed to support interoperable interaction over the world wide web.
The convergence service is invoked by an entity graph service.
The convergence service performs a conversion of data expressed with data definition ontologies (DDO) as available in the data sources to data expressed with the domain ontologies (DO) as used by the entity graphs and aggregates the resulting data.
The conversion service uses formal declarative rules for the conversion process.
An entity graph can be used as a data graph in the entity graph SPARQL endpoint to provide answers to queries on the named entity graph.
In order to be able to provide a user with a unified view of data from different data sources with each having different local semantics, an entity graph service is used that on demand produces an entity graph by specifying which data needs to be retrieved from identified data sources, invoking the convergence service to retrieve the data from the different data sources and convert the data from the local semantics to the domain ontology, and projecting the result to the model of the defined entity representation.

Entity graphs are constructed on demand based on the use case. These entity graphs are specific configurable entity representations with unification of data from different data sources.

The entity graph SPARQL endpoint may provide caching functionality to cache the generation of the entity representation.

The formal representation of an entity graph can be retrieved by resolving the URL of the named entity graph.

A specific ETL (Extract-Transform-Load) process can be defined for each of the targeted data consumer data schemas and the configured entity graphs.

The data warehouse exposes on demand domain entity graphs.

The data warehouse can be scaled at development time by allowing development of additional independent plug-ins to expose new entity graphs. Plug-ins for existing entity graphs do not need to be adapted.

If the temporal information extracted or retrieved from the data repository is not represented in a semantic representation, a step 2 is required to convert it into a semantic representation such as an RDF representation. Alternative applicable semantic representations are a conceptual graph representation or a topic maps.
E.g. year '2000' entered in a partial date is retrieved as an xsd long integer ("2000"^^xsd:long).

Preferably the formalized data are kept as close as possible to the original data, to achieve this goal preferably minimal data interpretation is performed.

Data are preferably retrieved on demand, just in time for use in an application such as clinical study.

Next, conversion rules are applied to the extracted semantic data.
Conversion rules are applied to convert the semantic representation of the temporal information into a formal representation. Thus time entities are generated which are represented with formal ontologies. E.g. A partial date is converted as an interval.

This conversion is preferably performed upon request.

The converted results could use existing ontologies to represent intervals and time points.

For example W3C Time ontology can be used as target ontology. The W3C Time ontology captures the widely used Allen Interval temporal concepts and provides an instant class to formalize timepoints.

If temporal information is not explicitly present in the data of the event, an additional step, as described furtheron, is required.

If the event does no comprise explicit temporal information, temporal information needs to be inferred from other temporal information available in the data record of the event. Temporal relationships, which are implicitly embedded in the source data, are explicitly expressed at this stage.

When the temporal information in all records required for a certain application, such as for a clinical decision making process, are converted into a formal representation, the converted data can be integrated with data from other sources to carry out temporal reasoning, resulting in knowledge on a relation between the events to which these temporal data pertain.

Temporal reasoning comprises first order reasoning such as Allen calculus, which defines possible relations between time intervals and provides a composition table that can be used as a basis for reasoning about temporal descriptions of events.

Temporal relationships such as before, during, overlaps, after, etc. are interpreted.

For example: An event may specify that medication was administered to a patient in January 2010. Suppose that the event in the electronic patient record further mentions a health problem (disease) in February 2010. The temporal information January 2010 as well as February 2010 is incomplete (partial) since only very rough date information is available.
The method of this invention is then applied to deduce explicit, complete temporal information with which further reasoning can be performed.

A first period from Jan. 1, 2010 to Jan 31, 2010 is known. In this period medication was administered.
In a second period from February 1, 2010 to February 28, 2010 the disease has occurred.
Temporal reasoning will result in the fact that the disease happened after the administration of the medication.

The method of the present invention is advantageous in that
- The method enables relating data records that have temporal data in incomplete form or that have the temporal data in different formats. It even provides that data can be used that do not have explicit temporal information in the record. The method meets the gap between time related data stored in an electronic health record and temporal data required for clinical research.
- It allows temporal reasoning between events.
- It furthermore allows a very flexible use of data. Data can be represented by an ontology that best fits a target application.
- The reliability of the conversion can be checked by means of a third party proof checker.

The present invention can be implemented as a computer program product adapted to carry out the steps set out in the description.

The computer executable program code adapted to carry out the steps set out in the description can be stored on a computer readable medium.

## Claims

1. A method for formalizing temporal data in an electronic patient record comprising the steps of
- (1) extracting data regarding at least one event from a data repository,
- (2) if the extracted data is not in a semantic representation, performing a syntactic transformation of extracted data from their original representation as extracted from said data repository into a first semantic representation,
- (3) converting extracted and transformed data into a representation with explicit semantics regarding temporal information.
- (4) if the extracted data comprises no explicit temporal data, deducing temporal data from other extracted data pertaining to said event and attaching deduced temporal data to the event data.

2. A method according to claim 1 wherein said first semantic representation is an RDF representation.

3. A method according to claim 1 wherein said data repository is a data warehouse.

4. A method according to claim 1 wherein said conversion is based on the application of N3 rules.

5. A method according to claim 1 wherein said explicit semantics depend on an ontology dictated by a target application in which said temporal data are used.

6. A method according to claim 1 wherein said partial data is converted to a time period represented with said ontology.

7. A method according to claim 1 wherein temporal reasoning is applied to the result of step 4 for at least two events.

8. A method according to claim 1 wherein said steps are performed on request.

9. A method according to claim 1 wherein interpretation of said other extracted data in order to deduce temporal data of an event is minimal so as to remain close to the original available information of an event.

10. A computer program product adapted to carry out the method of any of the preceding claims when run on a computer.

11. A computer readable medium comprising computer executable program code adapted to carry out the steps of any of claims 1 - 9.
